(19) Europäisches Patentamt
European Patent Office
Office européen des brevets

(11) EP 1 021 164 B1

(12) **EUROPÄISCHE PATENTSCHRIFT**

(45) Veröffentlichungstag und Bekanntmachung des Hinweises auf die Patenterteilung:
**27.02.2002 Patentblatt 2002/09**

(21) Anmeldenummer: **98965712.7**

(22) Anmeldetag: **28.11.1998**

(51) Int Cl.[7]: **A61K 7/48**

(86) Internationale Anmeldenummer:
**PCT/EP98/07701**

(87) Internationale Veröffentlichungsnummer:
**WO 99/29284 (17.06.1999 Gazette 1999/24)**

(54) **KOSMETISCHE MITTEL**

COSMETIC AGENTS

PRODUITS DE BEAUTE

(84) Benannte Vertragsstaaten:
**AT BE CH CY DE DK ES FI FR GB GR IE IT LI LU MC NL PT SE**

(30) Priorität: **08.12.1997 DE 19754282**

(43) Veröffentlichungstag der Anmeldung:
**26.07.2000 Patentblatt 2000/30**

(73) Patentinhaber: **Henkel Kommanditgesellschaft auf Aktien**
**40191 Düsseldorf (DE)**

(72) Erfinder:
- **HÖFFKES, Horst**
  **D-40595 Düsseldorf (DE)**
- **MAAK, Norbert**
  **D-40721 Hilden (DE)**
- **ROHLAND, Christa**
  **D-40468 Düsseldorf (DE)**

(56) Entgegenhaltungen:
**GB-A- 2 153 679** **US-A- 4 198 393**
**US-A- 5 453 276**

- **DATABASE WPI Section Ch, Week 9536 Derwent Publications Ltd., London, GB; Class D21, AN 95-271162 XP002108031 & IL 111 232 A (PROFESSOR OSCAR 9999 CO) , 30. März 1995**
- **DATABASE WPI Section Ch, Week 9809 Derwent Publications Ltd., London, GB; Class B04, AN 98-087406 XP002108032 & AU 28382 97 A (NINETEENTH MAYBARB PTY LTD), 15. Januar 1998**
- **DATABASE WPI Section Ch, Week 9226 Derwent Publications Ltd., London, GB; Class B04, AN 92-211987 XP002108033 & JP 04 139125 A (TAKASAGO PERFUMERY CO LTD), 13. Mai 1992**
- **DATABASE WPI Section Ch, Week 9828 Derwent Publications Ltd., London, GB; Class B07, AN 98-316669 XP002108034 & JP 10 114636 A (SUNSTAR CHEM IND CO LTD) , 6. Mai 1998**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 021 164 B1

Printed by Jouve, 75001 PARIS (FR)

## Beschreibung

Die Erfindung betrifft kosmetische Mittel enthaltend Caryophyllen, sowie die Verwendung von Caryophyllen zur Herstellung von Haut- und Haarpflegemitteln.

Die kosmetische Behandlung von Haut und Haaren ist ein wichtiger Bestandteil der menschlichen Körperpflege. Nicht zuletzt durch die starke Beanspruchung der Haut und Haare durch Umweltbelastungen nimmt die Bedeutung von Pflegeprodukten zu.

Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare zusätzlich in unerwünschter Weise beeinflussen können. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es wird daher seit langem empfohlen, die Haare in diesem Fall einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert. Derartige Mittel werden - zumeist mit relativ niedrigen Wirkstoffkonzentrationen - auch als im Haar verbleibende Präparate angeboten.

Weiterhin wurden in jüngster Zeit sogenannte Kombinationspräparate entwickelt, um den Aufwand der genannten mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können aber noch nicht alle Wünsche der Verbraucher erfüllen.

So ist beispielsweise die biologische Abbaubarkeit der quaternären Ammoniumverbindungen nicht voll befriedigend und ihre in vielen Fällen mangelnde Kompatibilität mit Aniontensiden schränkt die Formulierungsmöglichkeiten stark ein. Die gleichen Nachteile gelten auch für die kationischen Polymeren. Weiterhin können bei bestimmten Polymeren unerwünschte Kumulationen auf dem Haar auftreten.

Alkylphosphonate und Alkylphosphate sind zwar mit anionischen Tensiden kompatibel, verfügen aber nur über begrenzte pflegende Eigenschaften.

Auch die kosmetische Pflege der Haut hat in jüngster Zeit an Bedeutung gewonnen, da viele Menschen ihre Haut als empfindlich einstufen, andere durch häufige Einwirkung von Sonnenstrahlung oder von hautreizenden Agenzien ihre Haut belasten oder an allergischen und entzündlichen Zuständen der Haut erkrankt sind.

Es hat daher nicht an Versuchen gefehlt, durch kosmetische und dermatologische Hautbehandlungsmittel solchen Hautzuständen vorzubeugen oder diesen abzuhelfen. In diesem Zusammenhang ist beispielsweise der Einsatz von Panthenol und/oder Nerzöl in Hautbehandlungsmitteln bekannt.

Aus der Literatur ist die Verwendung von Caryophyllen in kosmetischen Bereichen bekannt, beispielsweise beschreibt das Dokument US 5,453,276 den Einsatz von Caryophyllen in kosmetischen Mitteln zur Erzielung einer antimikrobiellen Wirkung.

Die Anmeldung GB-2 153 679 nennt Caryophyllen als Bestandteile von Zahnpflegeprodukten, z. B. zur Farbstabilisierung.

Aus den oben genannten Gründen besteht jedoch im Haut- und Haarbereich weiterhin ein Bedarf nach Wirkstoffen bzw. Wirkstoffkombinationen für kosmetische Mittel mit guten pflegenden Eigenschaften und guter biologischer Abbaubarkeit, die sich problemlos in bestehende Rezepturen einarbeiten lassen.

Überraschenderweise wurde gefunden, daß die erfindungsgemäßen kosmetischen Mittel, enthaltend 0,1 bis 10, insbesondere 0,6 bis 5 Gew.-% Caryophyllen sich durch eine gute Haut- bzw. Haarverträglichkeit sowie pflegende Wirkung auszeichnen und insbesondere bei Einsatz in der Haarkosmetik zu einer verbesserten Naßkämmbarkeit sowie einem weicheren Griff des Haares führen. Insbesondere überraschend war, daß bei Verwendung von Caryophyllen in Haut- und Haarpflegeprodukten sehr gute Effekte bezüglich der Langzeitwirkung, insbesondere der verbesserten Naßkämmbarkeit sowie der Pflegewirkung, erzielt werden konnten.

Der Gegenstand der Erfindung betrifft die Verwendung von Caryophyllen in Haarpflegemitteln zur Verbesserung der Naßkämmbarkeit.

<u>Caryophyllen</u>

Caryophyllene sind aus etherischen Ölen, beispielsweise Gewürznelkenöl, isolierbare Sesquiterpene mit einem charakteristischen Geruch. Man findet sie auch in vielen weiteren ätherischen Ölen, so beispielsweise als Bestandteil von Copaiva-Balsam oder Zimtbaumöl. Inzwischen hat man herausgefunden, daß es sich bei den ursprünglich

als α-, β- und γ-Caryophyllen bezeichneten Substanzen nur bei dem α- und dem β-Caryophyllen um Verbindungen der gleichen Summenformel handelt. Heutzutage wird üblicherweise das β-Caryophyllen als Caryophyllen bezeichnet, während das α-Caryophyllen auch als Humulen bekannt ist. β-Caryophyllen wird bereits seit langem als Riechstoff eingesetzt. Im Sinne der vorliegenden Anmeldung soll unter Caryophyllen sowohl α-, als auch vorzugsweise β-Caryophyllen verstanden werden.

Im Sinne der vorliegenden Erfindung wird Caryophyllen bevorzugt in konzentrierter Form verwendet. Darunter ist im Rahmen der Lehre der Erfindung Caryophyllen mit einem Aktivsubstanzanteil von mindestens 60, vorzugsweise mindestens 90 Gew.-% zu verstehen. Es ist jedoch auch möglich, wie bereits aus dem Stand der Technik bekannt Caryophyllen enthaltende ätherische Öle zu verwenden. Bislang wurden diese Öle primär zur Aromatisierung der entsprechenden Mittel eingesetzt. Der vorliegenden Erfindung liegt jedoch die Erkenntnis zugrunde, daß insbesondere hochkonzentriertes Caryophyllen pflegende Eigenschaften für Haut und Haare besitzt.

Ein weiterer Gegenstand der Erfindung sind kosmetische Mittel, die 0,1 - 10 Gew.-% Caryophyllen und zusätzlich Fettalkohole enthalten.

## Fettalkohole

Unter Fettalkoholen sind primäre aliphatische Alkohole der Formel **(I)** zu verstehen,

$$R^1OH \qquad (I)$$

in der $R^1$ für einen linearen oder verzweigten Kohlenwasserstoffrest mit 6 bis 22 Kohlenstoffatomen steht, der gesättigt oder olefinisch ungesättigt mit bis zu 3 Doppelbindungen ist.

Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind langkettige Fettalkohole wie Cetylalkohol und Stearylalkohol, sowie technische Fettalkoholmischungen mit 12 bis 18 Kohlenstoffatomen wie beispielsweise Kokos-, Palm-, Palmkem- oder Talgfettalkohol.

Die erfindungsgemäßen Mittel enthalten die Fettalkohole vorzugsweise in Mengen von 0,1 bis 20, insbesondere 1 bis 10 Gew.-%.

## Tenside

In einer besonders bevorzugten Ausführungsform enthalten die erfindungsgemäßen Mittel neben Caryophyllen und Fettalkoholen weiterhin Tenside. Es kann sich dabei sowohl um nichtionische, anionische, kationische, zwitterionische als auch amphotere Tenside handeln; bevorzugt sind jedoch nichtionische und/ oder anionische Tenside, insbesondere bevorzugt sind Verbindungen mit einer oder mehreren Carboxylgruppen.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Fettalkoholsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte, Homologenverteilung aufweisen. Insbesondere bevorzugt im Sinne der vorliegenden Erfindung werden carboxygruppenhaltige Aniontenside, vorzugsweise Seifen, sowie Ethersulfate eingesetzt. Typische Beispiele für **nichtionische Tenside** sind Fettalkoholpolyglycolether, Alkylphenolpolyglycolether, Fettsäurepolyglycolester, Fettsäureamid-polyglycolether, Fettaminpolyglycolether, alkoxylierte Triglyceride, Mischether bzw. Mischformale, Alk(en)yloligoglykoside, Fettsäure-N-alkylglucamide, Polyolfettsäureester, Zuckerester, Sorbitanester, Polysorbate und Aminoxide.

Als nichtionogene Tenside im Sinne der vorliegenden Erfindung können vorzugsweise die folgenden Verbindungen eingesetzt werden:

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der

Alkylgruppe,

- $C_{12}$-$C_{22}$-Fettsäuremono- und -diester von Anlagerungsprodukten von 1 bis 30 Mol Ethylenoxid an Glycerin,
- $C_8$-$C_{22}$-Alkylmono- und oligoglycoside und deren ethoxylierte Analoga sowie
- Anlagerungsprodukte von 5 bis 60 Mol Ethylenoxid an Rizinusöl und gehärtetes Rizinusöl.

Bei diesen Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den nichtionogenen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Alkylpolyglykoside gemäß der Formel $RO\text{-}(Z)_x$, in der R steht für einen Alkylrest mit 8 bis 22 Kohlenstoffatomen, Z für einen Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R

- im wesentlichen aus $C_8$- und $C_{10}$-Alkylgruppen,
- im wesentlichen aus $C_{12}$- und $C_{14}$-Alkylgruppen,
- im wesentlichen aus $C_8$- bis $C_{16}$-Alkylgruppen oder
- im wesentlichen aus $C_{12}$- bis $C_{16}$-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfo-betaine.

Bei den genannten Tensiden handelt es sich ausschließlich um bekannte Verbindungen. Hinsichtlich Struktur und Herstellung dieser Stoffe sei auf einschlägige Übersichtsarbeiten, beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217,** verwiesen.

Die erfindungsgemäßen Mittel enthalten in einer bevorzugten Ausführungsform 0,1 bis 40, insbesondere 1 bis 30 Gew.-% an Tensid. Diese Mengen sind insbesondere davon abhängig, um welches kosmetische Mittel es sich handelt, so weisen Duschbäder üblicherweise einen höheren Tensidgehalt auf als beispielsweise Haarspülungen.

In einer weiteren bevorzugten Ausführungsform enthalten die erfindungsgemäß zu verwendenden Mittel noch Dicarbonsäuren ausgewählt aus der Gruppe, die gebildet wird durch Verbindungen der allgemeinen Formel (II),

(II)

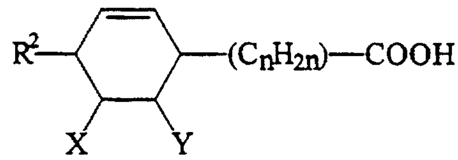

in der $R^2$ steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (II), die zusätzlich noch 1 bis 3 Methyl- oder Ethylsubstituenten am Cyclohexenring tragen, Dicarbonsäuren, die aus den Dicarbonsäuren gemäß Formel (II) formal durch Anlagerung eines Moleküls Wasser an die Doppelbindung im Cyclohexenring entstehen und Alkyl- sowie Alkenylbernsteinsäuren mit 1 bis 18 C-Atomen in der Alkylkette, oder einem physiologisch verträglichen Salz einer dieser Säuren

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-l-octansäure. Solche Verbindungen sind kommerziell unter den Bezeichnungen Westvaco Diacid® 1550 bzw. Diacid® H 240 (Hersteller: Westvaco), erhältlich.

Neben den erfindungsgemäßen Inhaltsstoffen können auch noch andere Komponenten enthalten sein. Die Auswahl dieser weiteren Komponenten wird im wesentlichen durch die Art des kosmetischen Mittels bestimmt.

Besonders bevorzugt werden zusätzlich **wasserlösliche Polymere** eingesetzt. Dabei kann es sich im Sinne der vorliegenden Erfindung sowohl um nichtionische, kationische, aionische und/ oder amphotere Polymere handeln. Bevorzugte Polymere sind die nichtionogenen Polymere wie beispielsweise Polyvinylpyrrolidon, Vinylpyrrolidon/Vinylacetat-Copolymere und Celluloseether, Stärke und/ oder Cyclodextrine sowie anionische Polymere wie beispielsweise die unter den Handelsnamen Carbopol® , Acrysol® und POC® (DEGUSSA) erhältlichen Acrylate.

Daneben können auch noch weitere **Fette und Öle** enthalten sein, beispielsweise natürliche Fette und Öle, Mono-, Di- und Triglyceride von gesättigten und ungesättigten Fettsäuren, Ethylenglykolester von Fettsäuren, Fettsäuren, niedrig ethoxylierte Fettsäuren, niedrig ethoxylierte Fettalkohole, Cholesterin und niedrig ethoxylierte Cholesterine, aber auch synthetische Produkte wie beispielsweise Silikonöle und deren Mischungen.

Die Art der erfindungsgemäßen kosmetischen Mittel unterliegt keinen Beschränkungen. Die erfindungsgemäßen Mittel können sowohl auf Haut und/ oder Haar verbleiben (leave-on), als auch, insbesondere bei Haarbehandlungsmitteln nach einer Einwirkzeit von wenigen Sekunden bis Minuten wieder ausgespült werden (rinse-off). Beispiele für erfindungsgemäß verwendete Mittel sind Duschlotionen, Duschbäder, Bodylotion, Handcreme, Shampoos, Spülungen, Kuren, Konditioniermittel, Tönungsmittel, Färbemittel, Dauerwellmittel, Fixiermittel, Haarsprays und Fönwellen.

Weitere Komponenten dieser Mittel können dann beispielsweise sein:

- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, $N_2O$, Dimethylether, $CO_2$ und Luft sowie
- Antioxidantien,
- Reduktionsmittel wie z.B. Thioglykolsäure und deren Derivate, Thiomilchsäure, Cysteamin, Thioäpfelsäure und α-Mercaptoethansulfonsäure,

Die erfindungsgemäßen Mittel können beispielsweise als wäßrige, alkoholische oder wäßrig-alkoholische Lösungen, Cremes, Gele oder Emulsionen formuliert sein.

**A. Untersuchungsverfahren**

A. 1 Gezielte Schädigung von Haarsträhnen

Trockene Haarsträhnen von ca. 2 g Gewicht (Fischbach und Miller Typ 6923) wurden einmal mit 8 g Blondiermischung (Marktprodukt Poly Blond Medium Aufheller) je Strähne eine halbe Stunde lang blondiert. Nach dem Auswaschen der Blondiermischung wurden die Haarsträhnen unmittelbar ohne Zwischentrocknung einer Dauerwellbehandlung mit dem Marktprodukt Poly Lock Normal, je 7g Wellgel bzw. Fixiermittel pro Strähne, unterzogen. Die Einwirkdauern von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

A.2 Prüfung der Naßkämmbarkeit

Die gespülte Haarsträhne wurde vor der Prüfung mit je 0,2 ml einer 50%igen Lösung von Texapon® N25 (28%ige Lösung von Natriumlaurylethersulfat in Wasser)-Lösung intensiv shampooniert und anschließend ausgespült. Danach wurden 0,5 ml der zu prüfenden Formulierung gleichmäßig in das Haar massiert, eine Minute im Haar belassen und dann sorgfältig ausgespült. Danach wurde mit einem feinzinkigen Kamm gekämmt und der Kämmwiderstand subjektiv beurteilt. Anschließend wurde an derselben Strähne in gleicher Weise eine Standardformulierung als Vergleich geprüft. Die Beurteilung wurde entsprechend einem Beurteilungssystem von 1 (= sehr gut) bis 5 (= ungenügend) abgegeben.

**B. Rezepturbeispiele**

**1. Cremeshampoo/ Duschbad**

Tab.1:

| **Cremeshampoo (Rezepturen 1,2)/ Duschbad (Rezepturen 3,4)** | | | | |
|---|---|---|---|---|
| **Rohstoff** | **Rezepturnummer** Angaben in Gew.-% | | | |
| | 1 | 2 | 3 | 4 |
| **Kokosfettalkohol** | 4,0 | 3,0 | 5,0 | 3,0 |
| **Texapon® N 28**[1] | 25,0 | 25,0 | 30,0 | 30,0 |
| **Na-Stearat** | - | - | 3,0 | 3,0 |
| **Caryophyllen 86 gew.-%ig** | - | 1,0 | - | 2,0 |
| **Wasser** | 71,0 | 71,0 | 62,0 | 62,0 |
| **[1] Laurylethersulfat Natriumsalz, 30 Gew.-% Aktivsubstanz** | | | | |

Für die Rezepturen 1 und 2 wurde die Naßkämmbarkeit wie bereits beschrieben bewertet, während für die Rezepturen 3 und 4 der Pflegeeffekt auf der Haut (ebenfalls subjektiv) beurteilt wurde.

Tab. 2:

| **Naßkämmbarkeit/ Pflegeeffekt** | |
|---|---|
| **Rezeptur** | **Bewertung** |
| 1 | 4 bis 5 |
| 2 | 3 |
| 3 | Nach dem Duschen kein Pflegeeffekt, trockene Haut |

Tab. 2: (fortgesetzt)

| Naßkämmbarkeit/ Pflegeeffekt | |
|---|---|
| **Rezeptur** | **Bewertung** |
| 4 | Geschmeidige gepflegte Haut |
| | nach dem Duschen |

**2. Haarspülungen**

Tab.3:

| Haarspülungen | | | | | | | | | | | |
|---|---|---|---|---|---|---|---|---|---|---|---|
| Rohstoff | Rezepturnummer Angaben in Gew.-% | | | | | | | | | | |
| | **5** | **6** | **7** | **8** | **9** | **10** | **11** | **12** | **13** | **14** | **15** |
| **Kokosfettalkohol** | 2,0 | 1,0 | 2,0 | 2,0 | - | - | - | - | - | - | - |
| **Cetearylalkohol** | - | - | - | - | - | 1 | 1 | 1 | 1 | 1 | - |
| **Texapon® N 28** | 1,6 | 1,6 | - | - | - | - | - | - | - | - | - |
| **Copaiva-Balsamöl** | - | 1,0 | - | 2,0 | - | - | - | - | - | - | - |
| **Cremophor® RH 40[2]** | - | - | - | - | - | - | - | - | 1 | 1 | - |
| **Saponin** | - | - | 0,4 | 0,4 | - | - | - | - | - | - | - |
| **Guarkernmehl** | - | - | 1,0 | 1,0 | 1,0 | 1,0 | - | - | - | - | - |
| **Sojalecithin** | - | - | - | - | 2,0 | 2,0 | - | - | - | - | - |
| **Candelilla-Wachs** | - | - | - | - | 3,0 | 2,0 | - | - | - | - | - |
| **Caryophyllen 86 gew.-%ig** | - | - | - | - | - | 1,0 | - | 0,5 | - | 0,5 | 0,5 |
| **Westvaco Diacid® H 240[3], 41 gew.-%ig** | - | - | - | - | - | - | 7,5 | 7,5 | - | - | 7,5 |
| **Citronensäure** | - | - | - | - | 2,0 | 2,0 | - | - | - | - | - |
| **Wasser** | 96,4 | 96,4 | 96,6 | 94,6 | 92 | 91 | 91,5 | 91,0 | 98,0 | 97,5 | 92,0 |

[2] Hydriertes Ricinusöl mit 45 EO (BASF)
[3] Kaliumsalz der 4-Hexyl-5(6)-carboxy-2-cyclohexen-1-octansäure (ca. 41 % Aktivsubstanz in Wasser) (WESTVACO)

Tab.: 4

| Naßkämmbarkeit | |
|---|---|
| **Rezeptur** | **Bewertung** |
| 5 | 4 bis 5 |
| 6 | 3 |
| 7 | 4 bis 5 |
| 8 | 3 |
| 9 | 3 |
| 10 | 2 |
| 11 | 2 |
| 12 | 1 bis 2 |

Tab.: 4 (fortgesetzt)

| Naßkämmbarkeit | |
|---|---|
| **Rezeptur** | **Bewertung** |
| 13 | 4 bis 5 |
| 14 | 3 bis 4 |
| 15 | 3 bis 4 |

**3. Färbecreme**

Tab.5:

| Färbecreme | | |
|---|---|---|
| **Rohstoff** | **Rezepturnummer** Angaben in Gew.-% | |
| | 16 | 17 |
| **Kokosfettalkohol** | 10,0 | 9,0 |
| **Tesapon® N 25** | 20,0 | 20,0 |
| **Dehyton® K**[4] | 10,0 | 10,0 |
| **Polymer JR**[5] | 0,4 | 0,4 |
| **Natriumsulfit** | 0,5 | 0,5 |
| **Ammoniumchlorid** | 0,4 | 0,4 |
| **Ammoniaklsg. 25 gew.-%ig** | 6,0 | 6,0 |
| **EDTA di Na-Salz** | 0,2 | 0,2 |
| **p-Aminophenolhydrochlorid** | 0,07 | 0,07 |
| **p-Toluylendiaminsulfat** | 0,2 | 0,2 |
| Resorcin | 0,09 | 0,09 |
| **Caryophyllen 86 gew.-%ig** | - | 1,0 |
| **Wasser** | 52,14 | 52,14 |

[4]N,N-Diethyl-N(C8/C18 kokosamidopropyl)ammoniumacetobetain (30 Gew.-% AS)
[5] Hydroxyethykellulose-Trimethylammoniumpropyl-Ether (Union Carbide)

Die Rezepturen gemäß Tabelle 5 wurden im Verhältnis 1:1 mit einer 6 gew.-% igen Wasserstoffperoxidlösung gemischt. Auf lichtblondem Harr wurde so eine mittelblonde Einfärbung erzielt. Die Prüfung auf Naßkämmbarkeit nach einer Einwirkzeit der Färbemischung von 30 Minuten führte nach dem Ausspülen zu einer Bewertung der Rezeptur 16 mit 3, und der Rezeptur 17 mit der subjektiven Note 2.

**4. Im Haar verbleibende Pflegelotion**

Tab.6:

| Pflegelotion | | |
|---|---|---|
| **Rohstoff** | **Rezepturnummer** Angaben in Gew.-% | |
| | **18** | **19** |
| **Ethanol (kosmetisch)** | 50,0 | **50,0** |

Tab.6: (fortgesetzt)

| Pflegelotion | | |
|---|---|---|
| **Rohstoff** | **Rezepturnummer** Angaben in Gew.-% | |
| | **18** | **19** |
| **Caryophyllen** | - | **1,0** |
| Wasser | 50,0 | 49,0 |

Je 0,5g der Pflegelotionen gemäß der Rezepturen 18 und 19 wurden in eine wie bereits beschrieben vorbehandelte feuchte Haarsträhne (2g) einmassiert. Anschließend wurde die Naßkämmbarkeit bestimmt. Die Rezeptur 18 wurde mit 5, die Rezeptur 19 hingegen mit 1 bewertet. Nach ein- bzw. zweimaligem Shampoonieren mit einem handelsüblichen Shampoo wurde erneut die Naßkämmbarkeit der beiden Haarsträhnen bestimmt. Die mit der Rezeptur 19 vorbehandelte Strähne erhielt nach einmaligem Shampoonieren die Bewertung 2 bis 3, nach zweimaligem Shampoonieren 3 bis 4.

**Patentansprüche**

1. Verwendung von Caryophyllen in Haarpflegemitteln zur Verbesserung der Naßkämmbarkeit.

2. Kosmetische Mittel enthaltend 0,1 bis 10 Gew.-% Caryophyllen sowie einen primären aliphatischen Alkohol der Formel (I)

$$R^1\,OH \qquad (I),$$

in der $R^1$ für einen linearen oder verzweigten Kohlenwasserstoffrest mit 6 bis 22 Kohlenstoffatomen steht, der gesättigt oder olefinisch ungesättigt mit bis zu 3 Doppelbindungen ist.

3. Kosmetische Mittel nach Anspruch 2, enthaltend 0,6 bis 5 Gew.-% Caryophyllen.

4. Kosmetische Mittel nach Anspruch 2 oder 3, enthaltend 1 bis 10 Gew.-% eines Alkohols der Formel (I).

5. Kosmetische Mittel nach einem der Ansprüche 2 bis 4, enthaltend einen Alkohol der Formel (I) mit 12 bis 18 C-Atomen.

6. Kosmetische Mittel nach einem der Ansprüche 2 bis 5, enthaltend anionische und/oder nichtionische Tenside.

7. Kosmetische Mittel nach Anspruch 6, enthaltend 1 bis 30 Gew.-% eines anionischen und/oder nichtionischen Tensids.

8. Kosmetische Mittel nach Anspruch 7, enthaltend ein Tensid mit einer oder mehreren Carboxylgruppen.

9. Verwendung von Mitteln nach einem der Ansprüche 2 bis 8 zur Pflege von Haut und/oder Haar.

10. Verwendung nach Anspruch 9, **dadurch gekennzeichnet, daß** es sich um ein im Haar verbleibendes Haarbehandlungsmittel handelt.

**Claims**

1. The use of caryophyllene in hair-care preparations for improving wet combability.

2. Cosmetic preparations containing 0.1 to 10% by weight of caryophyllene and a primary aliphatic alcohol corresponding to formula (I):

$$R^1OH \quad (I)$$

in which $R^1$ is a linear or branched $C_{6-22}$ hydrocarbon radical which is saturated or olefinically unsaturated with up to 3 double bonds.

**3.** Cosmetic preparations as claimed in claim 2 containing 0.6 to 5% by weight of caryophyllene.

**4.** Cosmetic preparations as claimed in claim 2 or 3 containing 1 to 10% by weight of an alcohol corresponding to formula (I).

**5.** Cosmetic preparations as claimed in any of claims 2 to 4 containing an alcohol corresponding to formula (I) with 12 to 18 carbon atoms.

**6.** Cosmetic preparations as claimed in any of claims 2 to 5 containing anionic and/or nonionic surfactants.

**7.** Cosmetic preparations as claimed in claim 6 containing 1 to 30% by weight of an anionic and/or nonionic surfactant.

**8.** Cosmetic preparations as claimed in claim 7 containing a surfactant with one or more carboxyl groups.

**9.** The use of the preparations claimed in any of claims 2 to 8 for skin and/or hair care.

**10.** The use claimed in claim 9, **characterized in that** the hair-care preparation is a leave-on preparation.

**Revendications**

**1.** Utilisation de caryophyllène dans des produits de soin des cheveux pour améliorer l'aptitude au peignage à l'état humide.

**2.** Produits cosmétiques contenant de 0,1 à 10 % en- poids de caryophyllène ainsi qu'un alcool aliphatique primaire de formule (I)

$$R^1OH \quad (I)$$

dans laquelle $R^1$ représente un radical hydrocarbure linéaire ou ramifié ayant de 6 à 22 atomes de carbone qui est saturé ou oléfiniquement insaturé avec jusqu'à 3 doubles liaisons.

**3.** Agent cosmétique selon la revendication 2, contenant de 0,6 à 5 % en poids de caryophyllène.

**4.** Agent cosmétique selon la revendication 2 ou 3, contenant de 1 à 10 % en poids d'un alcool de formule (I).

**5.** Agent cosmétique selon l'une des revendications 2 à 4, contenant un alcool de formule (I) ayant de 12 à 18 atomes de carbone.

**6.** Agent cosmétique selon l'une des revendications 2 à 5, contenant des agents tensioactifs anioniques et/ou non ioniques.

**7.** Agent cosmétique selon la revendication 6 contenant de 1 à 30 % en poids d'un agent tensioactif anionique et/ou non ionique.

**8.** Agent cosmétique selon la revendication 7 contenant un agent tensioactif comportant un ou plusieurs groupes carboxyle.

**9.** Utilisation d'agents selon l'une des revendications 2 à 8 pour le soin de la peau et/ou des cheveux.

**10.** Utilisation selon la revendication 9, **caractérisée en ce qu'** il s'agit d'un agent de traitement des cheveux restant dans la chevelure.